# EUROPÄISCHE PATENTSCHRIFT

(11) **EP 0 761 630 B1**
(45) Veröffentlichungstag und Bekanntmachung des Hinweises auf die Patenterteilung: **01.12.1999**
(21) Anmeldenummer: 96114060.5
(22) Anmeldetag: 03.09.1996
(51) Int. Cl.: C07C 37/07

(54) **Verfahren zur Herstellung von Alkylphenolen**
Process for preparing alkylphenols
Procédé de préparation d'alkylphénols

(30) Priorität: 12.09.1995 DE 19533665
(43) Veröffentlichungstag der Anmeldung: 12.03.1997
(73) Patentinhaber: BASF AKTIENGESELLSCHAFT, 67056 Ludwigshafen (DE)
(72) Erfinder: Bröcker, Franz Josef, Dr., 67061 Ludwigshafen (DE); Trübenbach, Peter, Dr., 67065 Ludwigshafen (DE); Baumann, Manfred, Dr., 68199 Mannheim (DE); Schubert, Jürgen, Dr., 67246 Dirmstein (DE)

(56) Entgegenhaltungen:
- EP-A- 0 123 233
- EP-A- 0 152 842

## Beschreibung

Die vorliegende Erfindung betrifft ein Verfahren zur Herstellung von Alkylphenolen durch katalytische Dehydrierung von Alkylcyclohex-2-en-1-onen bei erhöhten Temperaturen an Palladium/α-Al₂O₃-Trägerkatalysatoren, insbesondere von 2,3,6-Trimethylphenol aus 2,3,6-Trimethyl-cyclohex-2-en-1-on.

Aus der DE-A-16 68 874 ist die Umwandlung von 2,3,6-Trimethyl-2-cyclohexen-1-on durch Dehydrierung an Palladium-Trägerkatalysatoren bekannt. Als Träger sind Aluminiumoxid, Kieselsäure oder Aktivkohle beschrieben. In der Gasphase ist nur eine Dehydrierung an einem Pd/SiO₂-Katalysator beschrieben.

Führt man diese Dehydrierung mit den angeführten Katalysatoren in einer kontinuierlich arbeitenden Anlage durch, so zeigt sich, daß man nur kurzzeitig akzeptable Ergebnisse erzielt, da bereits nach wenigen Stunden die Katalysatoraktivität stark abnimmt. So ist es bisher nicht gelungen, mit diesen Katalysatoren längere Betriebszeiten zu fahren.

Der vorliegenden Erfindung lag daher die Aufgabe zugrunde, den zuvor genannten Nachteilen abzuhelfen, insbesondere einen Dehydrierkatalysator zu entwickeln, der bei hoher Ausbeute und Selektivität eine möglichst lange Standzeit ohne nennenswerten Aktivitätsabfall besitzt und dadurch ein wirtschaftlich arbeitendes Dehydrierverfahren ermöglicht.

Demgemäß wurde ein neues und verbessertes Verfahren zur Herstellung von Alkylphenolen der allgemeinen Formel I 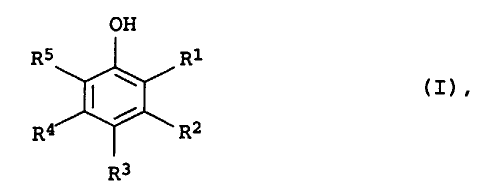 in der R¹, R², R³, R⁴ und R⁵ für Wasserstoff oder C₁- bis C₈-Alkyl stehen, durch Umsetzung von Alkyl-cyclohex-2-en-1-onen der allgemeien Formel II 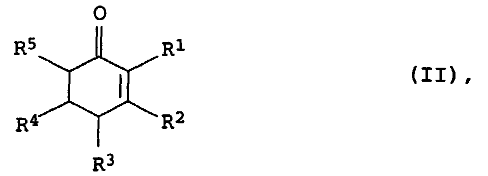 in der R¹, R², R³, R⁴ und R⁵ die oben genannten Bedeutungen haben, bei Temperaturen von 200 bis 400°C und Drücken von 0,01 bis 50 bar in Gegenwart von Palladium/α-Al₂O₃-Trägerkatalysatoren gefunden, welches dadurch gekennzeichnet ist, daß die Katalysatoren eine spezifische Oberfläche nach BET von 1 bis 10 m²/g, einen Porendurchmesser von 5 nm bis 300 µm und ein Porenvolumen von 0,1 bis 0,5 cm³/g aufweisen, wobei 80 bis 100 % der Poren einen Durchmesser von 40 bis 400 nm haben, sowie neue Palladium/α-Al₂O₃-Trägerkatalysatoren.

Das erfindungsgemäße Verfahren kann wie folgt durchgeführt werden:

Man kann das Alkyl-cyclohex-2-en-1-on II, bevorzugt dampfförmig (gasförmig), bei Temperaturen von 200 bis 400°C, bevorzugt 250 bis 350°C, besonders bevorzugt 280 bis 310°C und Drücken von 0,01 bis 50 bar, bevorzugt 0,1 bis 5 bar, besonders bevorzugt bei Normaldruck (Atmosphärendruck), bevorzugt in der Gasphase, über speziellen Palladium/α-Al₂O₃-Trägerkatalysator leiten. Vorteilhaft kann man dem Alkyl-cyclohex-2-en-1-on II in Dampfform vor dem Reaktionseingang ein Fremdgas, wie Stickstoff, Argon, Wasserdampf oder Wasserstoff, bevorzugt Wasserdampf oder Wasserstoff, besonders bevorzugt Wasserstoff zumischen, wobei man in der Regel ein Volumenverhältnis von Fremdgas zu dampfförmigen Alkyl-cyclohex-2-en-1-on II von 0,01:1 bis 100:1, bevorzugt 0,1:1 bis 10:1, besonders bevorzugt 0,7:1 bis 1,5:1 (etwa 1:1) wählt. Die Verweilzeiten am Katalysator betragen in der Regel 0,5 bis 50 Sekunden, vorzugsweise 2 bis 10 Sekunden.

Die speziellen erfindungsgemäßen Palladium/α-Al₂O₃-Trägerkatalysatoren weisen eine spezifische Oberfläche nach BET von 1 bis 10 m²/g, bevorzugt 4 bis 8 m²/g, einen Porendurchmesser von 5 nm bis 300 µm, bevorzugt 10 nm bis 100 µm, besonders bevorzugt 20 nm bis 50 µm und ein Porenvolumen von 0,1 bis 0,5 cm³/g, bevorzugt 0,2 bis 0,3 cm³/g auf, wobei 80 bis 100 %, bevorzugt 80 bis 98 %, besonders bevorzugt 80 bis 95 % der Poren einen Durchmesser von 40 bis 400 nm haben.

Die Herstellung der erfindungsgemäßen Palladium/α-Al₂O₃-Trägerkatalysatoren kann durch Herstellung des α-Al₂O₃-Trägers durch thermische Behandlung von γ-Al₂O₃-Pulver bei Temperaturen von 800 bis 1400°C erfolgen. Man erhält, je nach Calcinationstemperatur, ein α-Al₂O₃-Keramikpulver mit unterschiedlichen BET-Oberflächen und bimodalen Porenvolumenverteilungen. Das α-Al₂O₃-Keramikpulver hat in der Regel eine mittlere Partikelgröße von 0,3 bis 1,5 µm, vorzugsweise 0,5 bis 1 µm. Dieses Pulver wird in der Regel mittels eines Dispergators desagglomeriert und mit einem organischen Binder in einer Mischvorrichtung, beispielsweise einem Kneter, Extruder oder Scherwalzenextruder, eingearbeitet. Die Verformung kann durch Pressen oder Verstrangen erfolgen. Zur Entfernung des Bindersystems wird das verformte Produkt in der Regel an der Luft oder unter Inertgas bei 400 bis 600°C pyrolysiert und bei Temperaturen von 700 bis 1200°C, vorzugsweise 800 bis 1000°C calciniert. Durch Tränken mit einer Palladiumsalzlösung, wie Nitrat, Nitrosylnitrat, Halogenide, Carbonat, Carboxylate, Acetylacetonat, Chlorokomplexe, Nitritokomplexe oder Aminkomplexe, bevorzugt eine Palladiumnitratlösung, und anschließender Trocknung bei 200°C wird der erfindungsgemäße Dehydrierkatalysator erhalten. Der so erhaltene Katalysator kann in einen adiabatischen Schachtreaktor oder einen Röhrenreaktor eingefüllt und durch Überleiten von Wasserstoff mit einer Belastung von in der Regel 10 bis 100 Nl/l_{Kat.}x h und Erhitzen auf Temperaturen von 200 und 400°C aktiviert werden. Diese Katalysatoraktivierung dauert im allgemeinen 1 bis 24 Stunden, vorzugsweise 3 bis 6 Stunden.

Als Dispergatoren eignen sich beispielsweise Polyethylenglykol, Polypropylenglykol, Polybutandiolformal, Phthalsäureester, Ethylen-Vinylacetat-Copolymere, Montanesterwachse oder Stearinsäure.

Als organische Binder eignen sich beispielsweise Polyacetal, bevorzugt Polyoximethylen, Polyoxipropylen, Polyethylen, Polystyrol, Polymethylmethacrylat, Polyethylenoxid, Ethylenvinylacetat oder deren Gemische.

Die Substituenten R¹, R², R³, R⁴ und R⁵ in den Verbindungen I und II haben folgende Bedeutungen:
R¹,R²,R³,R⁴,R⁵
- Wasserstoff oder
- C₁- bis C₈-Alkyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl.

Alkylphenole I, insbesondere 2,3,6-Trimethylphenol eignen sich als Vorprodukt für die Synthese von Tocopherol (Vitamin E), Karrer, P. et al., Helv. Chim. Acta 21, 939 (1938), Ullmann (4.), 23, 643ff.

### Beispiele

### Beispiel 1

### a) Herstellung des Katalysatorträgers

1 kg α-Al₂O₃-Pulver (CT 3000 SG, der Fa. Alcoa) mit einer mittleren Korngröße von 0,7 µm wurden mit 162 g Polyacetal, einem Copolymer aus Trioxan und 2,5 % Butandiolformal, Molmasse 150.000, 41 g Polybutandiolformal, Molmasse 50.000, sowie 50 g Polyethylenglykol, Molmasse 800, versetzt und bei 180°C 1 verknetet. Das so erhaltene grobe Granulat wurde auf einem auf 180°C aufgeheizten Walzenstuhl mit 2 Walzen aufgegeben und zu einem 2 bis 3 mm dicken "Fell" gepreßt, in einer Messermühle zu einem quaderförmigen Granulat verarbeitet und unter Luft in einem Drehrohrofen bei 900°C 2 h pyrolysiert.

Der so erhaltene α-Al₂O₃-Träger besaß eine spezifische Oberfläche von 5,17 m²/g, ein Porendurchmesser von 300 µm bis 5 nm und ein Porenvolumen von 0,225 cm³/g. 83 % der Porendurchmesser lagen im Bereich von 40 bis 400 nm.

### b) Herstellung des Palladium/α-Al₂O₃-Katalysators

In einer Dragiertrommel wurde 1 kg des gemäß Beispiel 1a) erhaltenen α-Aluminiumoxidträgers mit einer Mischung aus 18,18 g Palladiumnitratlösung (11 Gew.-% Pd) und 210 g destilliertem Wasser versetzt und solange bewegt, bis die Lösung quantitativ aufgenommen war. Das getränkte Material wurde 10 Stunden bei 110°C getrocknet und anschließend 6 Stunden bei 200°C getempert.

### c) Aktivierung des Katalysators

100 ml des gemäß Beispiel 1b) hergestellten Katalysators wurden in einen Rohrreaktor mit 21 mm innerem Durchmesser eingefüllt und mit 10 Liter H2/h beaufschlagt. Innerhalb von 12 h wurde der Reaktor auf die Reaktionstemperatur von 300°C aufgeheizt.

### d) Herstellung von 2,3,6-Trimethylphenol

Nach Erreichen der Reaktionstemperatur wurde die Wasserstoffmenge auf 4 l/h reduziert und über einen Verdampfer gasförmiges 2,3,6-Trimethyl-cyclohex-2-en-1-on zudosiert. Am Ausgang des Reaktors wurde das feste 2,3,6-Trimethylphenol in einer Ausbeute und Selektivität von 99,6 % abgeschieden und analysiert. Man erhielt bei einer Belastung von 0,28 kg 2,3,6-Trimethyl-cyclohex-2-en-1-on/l_{Kat} x h und einer Reaktionstemperatur von 300°C einen Umsatz von 100 %.

### Vergleichsbeispiel A

Aluminiumoxidträgermaterial (Norton T 484) mit einer spezifischen Oberfläche nach BET von 4,1 m²/g und einem Porenvolumen von 0,406 cm³ im Porendurchmesserbereich von 300 µm bis 5 nm, bei dem 30 % einen Porendurchmesser zwischen 40 bis 400 nm haben, wurde wie in Beispiel 1b) mit Pd-Nitratlösung getränkt, so daß ein Pd-Katalysator mit 0,2 Gew.-% Pd erhalten wurde.

100 ml dieses Katalysators wurden wie in Beispiel 1c) beschrieben, in einen Rohrreaktor gefüllt, aktiviert und bei 300°C mit 2,3,6-Trimethyl-2-cyclohexen-1-on, analog Beispiel 1d), beaufschlagt. Bei einer Belastung von 0,280 kg/l_{Kat} x h wurde bei 100 %igem Umsatz eine Ausbeute und Selektivität von 92 % erzielt.

### Vergleichsbeispiel B

100 ml eines Pd-Tränkkatalysators, der mit einem Aluminiumoxidträger mit einer spezifischen Oberfläche nach BET von 0,98 m²/g und einem Porenvolumen im Porendurchmesserbereich von 300 µm bis 5 nm von 0,442 cm³/g, das quantitativ oberhalb von 400 nm Porendurchmesser liegt, hergestellt wurde, ergab bei der analog zum Beispiel 1d) durchgeführten Dehydrierung von 2,3,6-Trimethyl-2-cyclohexen-1-on bei 300°C und einer Belastung von 0,280 kg/l_{Kat} x h einen Umsatz von 100 % und eine Ausbeute und Selektivität von 83 %.

## Patentansprüche

1. Verfahren zur Herstellung von Alkylphenolen der allgemeinen Formel I 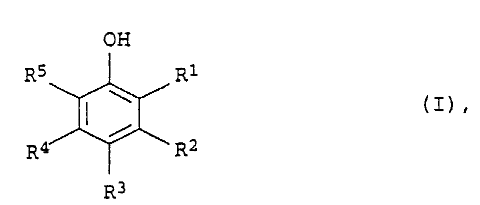 in der R¹, R², R³, R⁴ und R⁵ für Wasserstoff oder C₁- bis C₈-Alkyl stehen, durch Umsetzung von Alkyl-cyclohex-2-en-1-onen der allgemeien Formel II 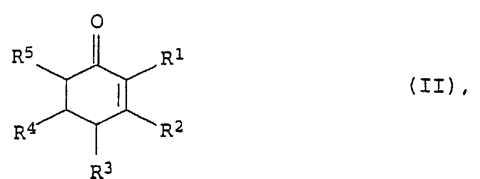 in der R¹, R², R³, R⁴ und R⁵ die oben genannten Bedeutungen haben, bei Temperaturen von 200 bis 400°C und Drücken von 0,01 bis 50 bar in Gegenwart von Palladium/α-Al₂O₃-Trägerkatalysatoren, dadurch gekennzeichnet, daß die Katalysatoren eine spezifische Oberfläche nach BET von 1 bis 10 m²/g, einen Porendurchmesser von 5 nm bis 300 µm und ein Porenvolumen von 0,1 bis 0,5 cm³/g aufweisen, wobei 80 bis 100 % der Poren einen Durchmesser von 40 bis 400 nm haben.

2. Verfahren zur Herstellung von Alkylphenolen I nach Anspruch 1, dadurch gekennzeichnet, daß die Palladium/α-Al₂O₃-Trägerkatalysatoren eine spezifische Oberfläche nach BET von 4 bis 8 m²/g aufweisen.

3. Verfahren zur Herstellung von Alkylphenolen I nach Anspruch 1, dadurch gekennzeichnet, daß die Umsetzung in der Gasphase durchführt.

4. Verfahren zur Herstellung von Alkylphenolen I nach Anspruch 1, dadurch gekennzeichnet, daß die Palladium/α-Al₂O₃-Trägerkatalysatoren aus einem α-Al₂O₃-Träger bestehen, der aus einem α-Al₂O₃-Pulver mit einer Korngröße von 0,3 bis 1,5 µm, einem Dispergator und einem organischen Binder durch Verformen, Pyrolyse und Calcinieren hergestellt wird.

5. Verfahren zur Herstellung von Alkylphenolen I nach Anspruch 1, dadurch gekennzeichnet, daß die Palladium/α-Al₂O₃-Trägerkatalysatoren aus einem α-Al₂O₃-Träger bestehen, der aus einem α-Al₂O₃-Pulver mit einer Korngröße von 0,5 bis 1 µm, einem Dispergator und einem organischen Binder durch Verformen, Pyrolyse und Calcinieren hergestellt ist.

6. Verfahren zur Herstellung von Alkylphenolen I nach Anspruch 1, dadurch gekennzeichnet, daß die Palladium/α-Al₂O₃-Trägerkatalysatoren durch Tränken des α-Al₂O₃-Trägers mit einer Palladiumnitratlösung und Trocknung bis 200°C hergestellt sind.

7. Verfahren zur Herstellung von Alkylphenolen I nach Anspruch 1, dadurch gekennzeichnet, daß man die Palladium/α-Al₂O₃-Trägerkatalysatoren bei 250 bis 330°C mit Wasserstoff aktiviert.

8. Verfahren zur Herstellung von Alkylphenolen I nach Anspruch 1, dadurch gekennzeichnet, daß R¹, R² und R⁵ für Methyl und R³ und R⁴ für Wasserstoff stehen.

9. Palladium/α-Al₂O₃-Trägerkatalysatoren, dadurch gekennzeichnet, daß die Katalysatoren eine spezifische Oberfläche nach BET von 1 bis 10 m²/g, einen Porendurchmesser von 5 nm bis 300 µm und ein Porenvolumen von 0,1 bis 0,5 cm³/g aufweisen, wobei 80 bis 100 % der Poren einen Durchmesser von 40 bis 400 nm haben.

10. Palladium/α-Al₂O₃-Trägerkatalysatoren nach Anspruch 9, dadurch gekennzeichnet, daß die Katalysatoren aus einem α-Al₂O₃-Träger bestehen, der aus einem α-Al₂O₃-Pulver mit einer Korngröße von 0,3 bis 1,5 µm, einem Dispergator und einem organischen Binder durch Verformen, Pyrolyse und Calcinieren hergestellt sind.

## Claims

1. A process for the preparation of alkylphenols of the formula I 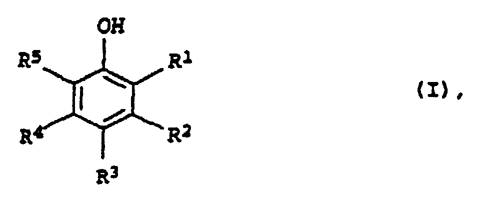 in which R¹, R², R³, R⁴ and R⁵ are hydrogen or C₁- to C₈-alkyl, by reacting alkylcyclohex-2-en-1-ones of the formula II 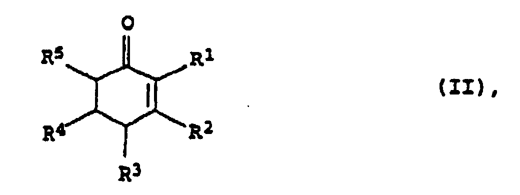 in which R¹, R², R³, R⁴ and R⁵ are as defined above, at temperatures of from 200 to 400°C and pressures of from 0.01 to 50 bar in the presence of α-Al₂O₃-supported palladium catalyst(s) catalysts, wherein the catalysts have a BET specific surface area of from 1 to 10 m²/g, a pore diameter of from 5 nm to 300 µm and a pore volume of from 0.1 to 0.5 cm³/g, from 80 to 100% of the pores having a diameter of from 40 to 400 nm.

2. A process for the preparation of alkylphenols I as claimed in claim 1, wherein the α-Al₂O₃-supported palladium catalysts have a BET specific surface area of from 4 to 8 m²/g.

3. A process for the preparation of alkylphenols I as claimed in claim 1, which comprises carrying out the reaction in the gaseous phase.

4. A process for the preparation of alkylphenols I as claimed in claim 1, wherein the α-Al₂O₃-supported palladium catalysts consist of an α-Al₂O₃ support which has been prepared from an α-Al₂O₃ powder having a particle size of from 0.3 to 1.5 µm, a dispersant and an organic binder by shaping, pyrolysis and calcination.

5. A process for the preparation of alkylphenols I as claimed in claim 1, wherein the α-Al₂O₃-supported palladium catalysts consist of an α-Al₂O₃ support which is prepared from an α-Al₂O₃ powder having a particle size of from 0.5 to 1 µm, a dispersant and an organic binder by shaping, pyrolysis and calcination.

6. A process for the preparation of alkylphenols I as claimed in claim 1, wherein the α-Al₂O₃-supported palladium catalysts are prepared by impregnating the α-Al₂O₃ support with a palladium nitrate solution and drying up to 200°C.

7. A process for the preparation of alkylphenols I as claimed in claim 1, wherein the α-Al₂O₃-supported palladium catalysts are activated with hydrogen at from 250 to 330°C.

8. A process for the preparation of alkylphenols I as claimed in claim 1, wherein R¹, R² and R⁵ are methyl, and R³ and R⁴ are hydrogen.

9. An α-Al₂O₃-supported palladium catalyst which has a BET specific surface area of from 1 to 10 m²/g, a pore diameter of from 5 nm to 300 µm and a pore volume of from 0.1 to 0.5 cm³/g, from 80 to 100% of the pores having a diameter of from 40 to 400 nm.

10. An α-Al₂O₃-supported palladium catalyst as claimed in claim 9, which consists of an α-Al₂O₃ support which has been prepared from an α-Al₂O₃ powder having a particle size of from 0.3 to 1.5 µm, a dispersant and an organic binder by shaping, pyrolysis and calcination.

## Revendications

1. Procédé pour la préparation d'alkylphénols de formule générale I 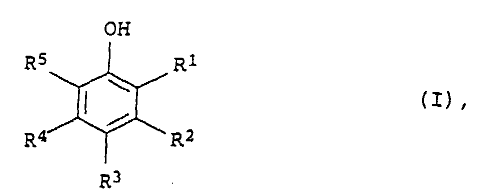 où R¹, R², R³, R⁴ et R⁵ désignent l'hydrogène ou un alkyle en C₁ à C₈, par réaction d'alkyl-cyclohex-2-ène-1-ones de formule générale II 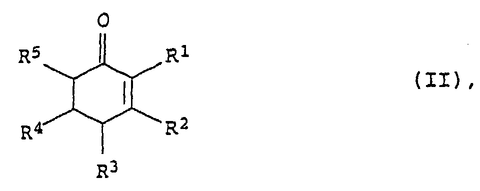 où R¹, R², R³, R⁴ et R⁵ ont les significations indiquées plus haut, à des températures de 200 à 400°C sous des pressions de 0,01 à 50 bar en présence de catalyseurs au palladium/support d'α-Al₂O₃, caractérisé par le fait que les catalyseurs ont une surface spécifique selon BET de 1 à 10 m²/g, un diamètre des pores de 5 nm à 300 µm et un Volume des pores de 0,1 à 0,5 cm³/g, tandis que 80 à 100% des pores ont un diamètre de 40 à 400 nm.

2. Procédé pour la préparation d'alkylphénols I selon la revendication 1, caractérisé par le fait que les catalyseurs au palladium/support d'α-Al₂O₃ présentent une surface spécifique selon BET de 4 à 8 m²/g.

3. Procédé pour la préparation d'alkylphénols I selon la revendication 1, caractérisé en ce que l'on effectue la réaction en phase gazeuse.

4. Procédé pour la préparation d'alkylphénols I selon la revendication 1, caractérisé par le fait que les catalyseurs au palladium/support d'α-Al₂O₃ consistent en un support d'α-Al₂O₃ qui est préparé à partir d'une poudre d'α-Al₂O₃ ayant une granulométrie de 0,3 à 1,5 µm, un agent dispersant et un liant organique par façonnage, pyrolyse et calcination.

5. Procédé pour la préparation d'alkylphénols I selon la revendication 1, caractérisé par le fait que les catalyseurs au palladium/support d'α-Al₂O₃ consistent en un support d'α-Al₂O₃ qui est préparé à partir d'une poudre d'α-Al₂O₃ ayant une granulométrie de 0,5 à 1 µm, un agent dispersant et un liant organique par façonnage, pyrolyse et calcination.

6. Procédé pour la préparation d'alkylphénols I selon la revendication 1, caractérisé par le fait que les catalyseurs au palladium/support d'α-Al₂O₃ sont préparés par imprégnation du support d'α-Al₂O₃ avec une solution de nitrate de palladium et séchage à 200°C.

7. Procédé pour la préparation d'alkylphénols I selon la revendication 1, caractérisé par le fait qu'on active les catalyseurs au palladium/support d'α-Al₂O₃ entre 250 et 330°C avec de l'hydrogène.

8. Procédé pour la préparation d'alkylphénols I selon la revendication 1, caractérisé par le fait que R¹, R² et R⁵ représentent un méthyle, tandis que R³ et R⁴ désignent l'hydrogène.

9. Catalyseurs au palladium/support d'α-Al₂O₃, caractérisés en ce que les catalyseurs ont une surface spécifique selon BET de 1 à 10 m²/g, un diamètre des pores de 5 nm à 300 µm et un volume des pores de 0,1 à 0,5 cm³/g, tandis que 80 à 100% des pores ont un diamètre de 40 à 400 nm.

10. Catalyseurs au palladium/support d'α-Al₂O₃ selon la revendication 9, caractérisés par le fait que les catalyseurs consistent en un support d'α-Al₂O₃ qui est préparé à partir d'une poudre d'α-Al₂O₃ ayant une granulométrie de 0,3 à 1,5 µm, un agent dispersant et un liant organique par façonnage, pyrolyse et calcination.
